(19) Europäisches Patentamt
European Patent Office
Office européen des brevets

(11) Publication number: **0 014 425**
**B1**

(12) **EUROPEAN PATENT SPECIFICATION**

(45) Date of publication of patent specification: **02.06.82**

(51) Int. Cl.³: **C 07 H 15/24,**
A 61 K 31/70

(21) Application number: **80100447.4**

(22) Date of filing: **29.01.80**

(54) **Anthracycline glycosides, methods for the production thereof and therapeutical compositions containing same.**

(30) Priority: **02.02.79 GB 7903700**

(43) Date of publication of application:
**20.08.80 Bulletin 80/17**

(45) Publication of the grant of the patent:
**02.06.82 Bulletin 82/22**

(84) Designated Contracting States:
**AT BE CH DE FR GB IT NL SE**

(56) References cited:
**CHEMICAL ABSTRACTS, Vol. 90, No. 3 15th January 1979, page 709, No. 23591r Columbus, Ohio, USA**
**EL KHADEM et al.: "Synthesis of 2-deoxy-L-fucopyranosyl-e-pyrromycinone and 2-deoxy-D-erythropentopyranosyldaunomycinone, -carminomycinone, and e-pyrromycinone"**

(73) Proprietor: **FARMITALIA CARLO ERBA S.p.A.**
**Via Carlo Imbonati n.24**
**I-20159 Milan (IT)**

(72) Inventor: **Cassinelli, Guiseppe**
**Via G. Matteotti n. 13**
**Voghera (Pavia) (IT)**
Inventor: **Arcamone, Federico**
**Via 4 Novembre n. 26**
**Nerviano (Milano) (IT)**
Inventor: **di Marco, Aurelio, Dr.**
**Via Plinio n. 59**
**Milano (IT)**

(74) Representative: **Hansen, Bernd, Dr.rer.nat. et al,**
**Hoffmann . Eitle & Partner Patentanwälte**
**Arabellastrasse 4**
**D-8000 München 81 (DE)**

**EP 0 014 425 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Anthracycline glycosides, methods for the production thereof and therapeutical compositions containing same

The invention relates to anthracycline glycosides, methods for the production thereof and therapeutical compositions containing same.

The invention provides anthracycline glycosides of the general formula I

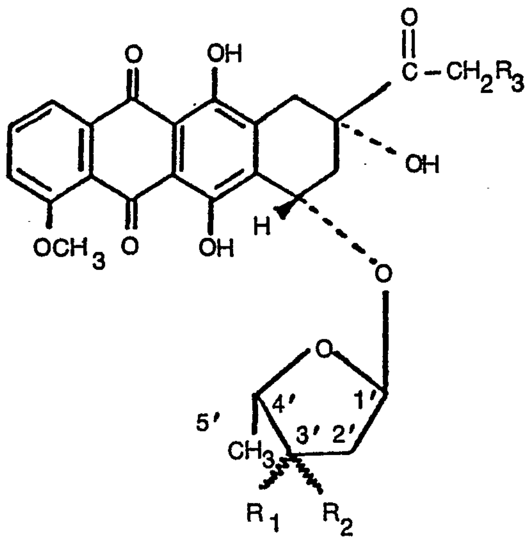

I

wherein one of $R_1$ and $R_2$ represents a hydrogen atom, the other of $R_1$ and $R_2$ represents a formyl, hydroxymethyl or aminomethyl group, and $R_3$ represents a hydrogen atom or a hydroxy or acyloxy group, and pharmaceutically acceptable acid addition salts of such compounds in which one of $R_1$ and $R_2$ represents an aminomethyl group.

The anthracycline glycosides of the general formula I, which contain branched-chain deoxy- or aminodeoxy-sugars, may be prepared from known anthracycline glycosides of the general formula II,

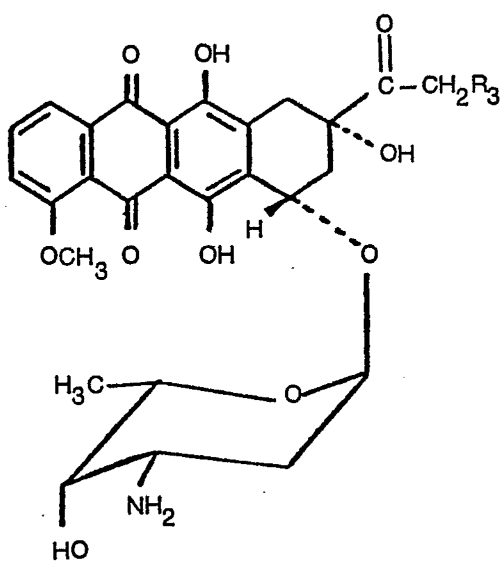

II

wherein $R_3$ has the meaning above described, by deamination and C—3′ epimerisation and, in the case of certain of the compounds, isomerisation and/or one of selective reduction and selective reductive amination. The anthracycline glycoside starting compound II in which $R_3$ represents a hydrogen atom is daunorubicin, that in which $R_3$ represents a hydroxy group is doxorubicin and those in which $R_3$ represents an acyloxy group are 14-O-acyl derivatives of doxorubicin.

The invention accordingly provides a method for the preparation of compounds of the general formula I in which one of $R_1$ and $R_2$ represents a hydrogen atom, the other of $R_1$ and $R_2$ represents a

formyl group and $R_3$ has the meaning above described, the method comprising reacting a compound of the general formula II with sodium nitrite in a cold aqueous acidic medium. This gives the desired new glycosides containing a 2,3,5-trideoxy-3′-C-formyl-$\chi$-L-pentofuranosyl moiety, as a result of a migration of C—5′ on C—3′ of a "diazotized intermediate", with consequent sugar ring contraction and formation of a 3′-C-formyl branched-chain (as described for 3-amino-3-deoxy-glycosides by J. M. William, in "Advances in Carbohydrate Chemistry and Biochemistry", vol. *31,* p. 28, 1975, Academic Press, New York, San Francisco and London).

The compounds of the general formula I in which one of $R_1$ and $R_2$ represents a hydrogen atom and the other of $R_1$ and $R_2$ represents a hydroxymethyl group whereas $R_3$ has the meaning above described may be obtained by the further step, within the process of the invention, of selective reduction. The reduction may be effected with sodium cyanoborohydride in an acidic buffer, suitably a 4:1 mixture of dioxan:aqueous acetate at pH 4.6, or more preferably with sodium triacetoxyborohydride in benzene according to the method described by G. W. Gribble and D. C. Ferguson in J.C.S. Chem. Comm. *1975,* p. 535.

The compounds of the general formula I in which one of $R_1$ and $R_2$ represents a hydrogen atom and the other of $R_1$ and $R_2$ represents an aminomethyl group whereas $R_3$ has the meaning above described may be obtained by the alternative further step, within the process of the invention, of selective reductive amination with sodium cyanoborohydride in the presence of methanolic ammonium acetate and a dehydrating agent. Preferably Molecular Sieve (Merck) is used as the dehydrating agent.

These reactions are set out in the following reaction scheme:

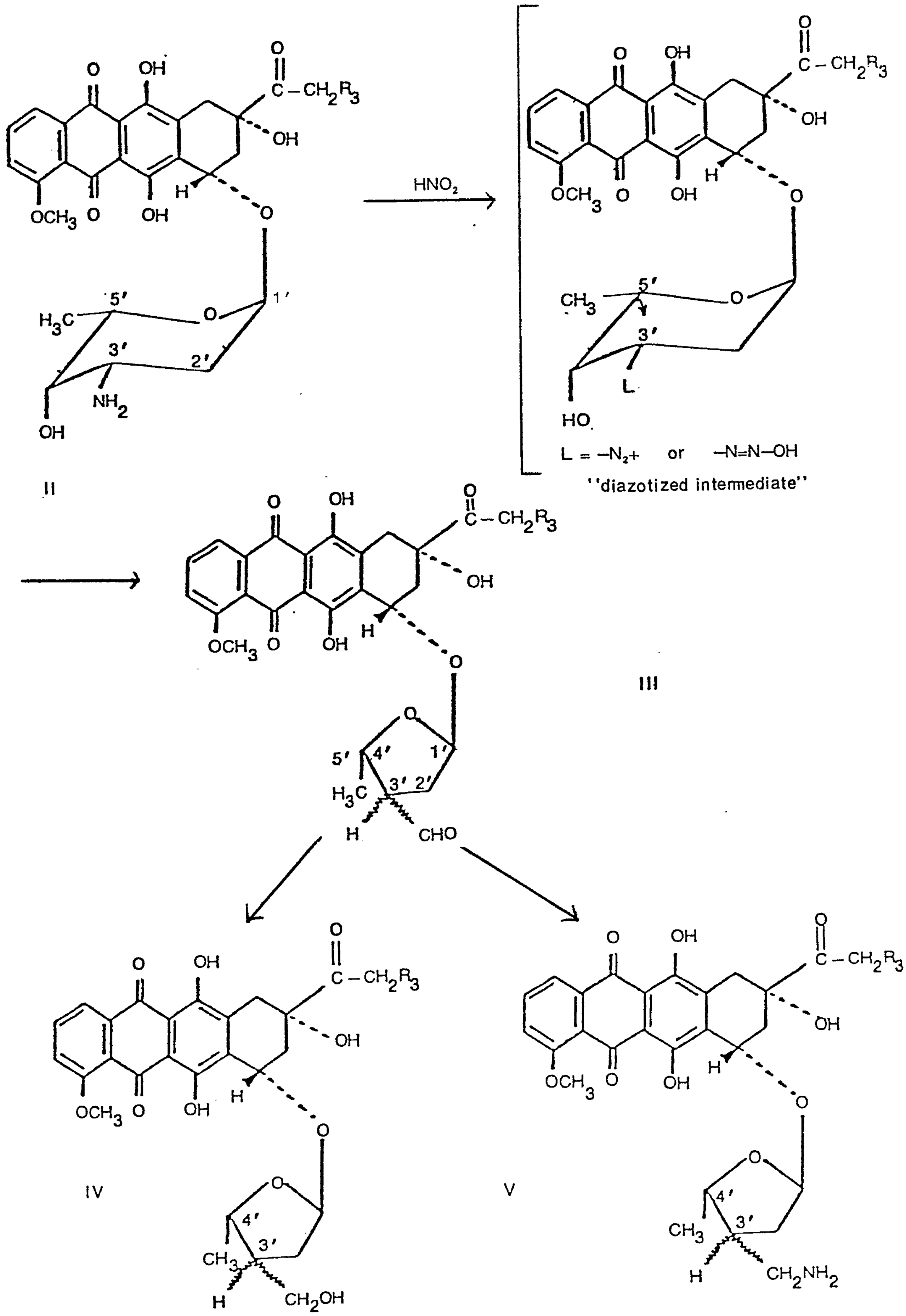


II

III

IV

V

In the case in which the compound of the general formula II is daunorubicin (II, $R_3$=H) only one (IIIa) of the two theoretically possible C-3′-epimers is obtained:

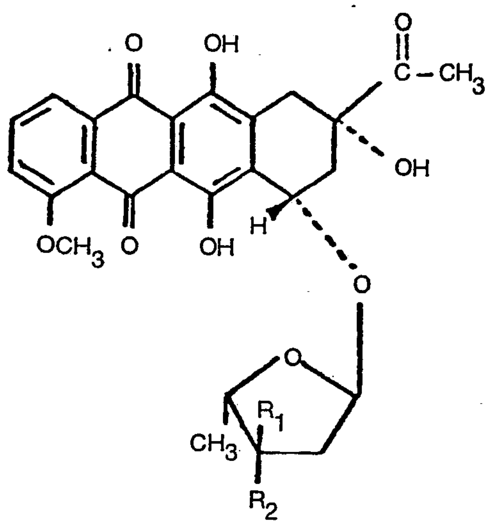

III-a ($R_1$=H, $R_2$=CHO)

This can be converted in the other (III-b)

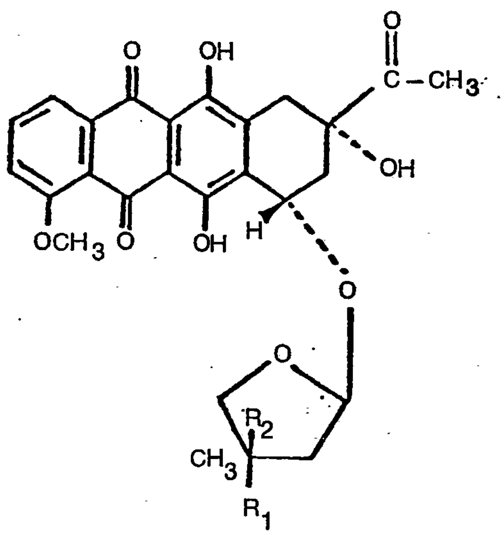

III-b ($R_1$=H, $R_2$=CHO)

by passing a chloroform solution of compound III-a through a column of silica gel buffered at pH 7.

The following Examples, in which all temperatures are expressed in degrees Centigrade, illustrate the invention.

BIOLOGICAL ACTIVITY

The cytotoxic activity of the new compounds have been tested against HeLa cells *"in vitro"* (time of exposure to the drugs: 24 Hrs) in comparison with daunorubicin and doxorubicin. The results are shown in Table 1.

TABLE 1 – Effect on HeLa cells viability "in vitro"

| Compound | $ID_{50}$ (ng/ml) |
|---|---|
| Daunorubicin . HCl | 7.4 |
| Compound IIIa (IMI–92) | 320 |
| Compound III–b (IMI–100) | 1.750 |
| Compound IV ($R_3$ = H) (IMI–102) | 1.600 |
| Doxorubicin . HCl | 10 |
| Compound III (R = OH) (IMI–99) | 140 |

The antitumor activity of the new compounds against P—388 leukemia in mice are reported in Table 2.

Compounds III-a, IV ($R_3$=H) and III ($R_3$=OH) at the tolerated doses (100~200 mg/Kg) display an antitumor activity higher or comparable to those of daunorubicin and doxorubicin.

TABLE 2 – Effect against P–388 leukemia in mice

| Compound | Dose [(a)] mg/Kg | T/C % | Toxic Deaths [(b)] |
|---|---|---|---|
| Daunorubicin . HCl [(c)] | 2.9 | 169 | 0/37 |
| | 4.4 | 167 | 0/38 |
| | 6.6 | 157 | 19/38 |
| III–a (IMI–92) [(d)] | 50 | 168 | 0/8 |
| | 100 | 163 | 0/5 |
| | 200 | 190 | 2/5 |
| III–b (IMI–100) [(e)] | 50 | 110 | 0/10 |
| | 100 | 110 | 0/10 |
| | 200 | 125 | 0/9 |
| IV ($R_3$ = H) (IMI–102) [(e)] | 50 | 130 | 0/7 |
| | 100 | 160 | 0/6 |
| | 200 | 220 | 0/5 |
| Doxorubicin . HCl [(c)] | 4.4 | 195 | 0/17 |
| | 6.6 | 206 | 0/17 |
| | 10.0 | 244 | 4/18 |
| III–($R_3$ = OH) (IMI–99) [(e)] | 50 | 163 | 0/9 |
| | 100 | 190 | 0/9 |
| | 200 | 231 | 1/7 |

(a) Mice were treated i.p. on day 1 after tumor cell inoculation.
(b) Evaluated on the base of the microscopic autoptic findings.
(c) Injected in water solution.
(d) Injection in 5% aqueous ethanol suspension.
(e) Injected in 1:9 Tween 80 : water suspension.

Example 1

Preparation of 7-O-(2,3,5-trideoxy-3-C-formyl-$\alpha$-L-threopentofuranosyl)-daunomycinone (III-a) (IMI—92).

A solution of daunorubicin hydrochloride (II: $R_3$=H; 4.5 g, 8 mmol) in water (200 ml) was cooled to 0° then treated with sodium nitrite (2.76 g, 80 mmol) and 1 N aqueous acetic acid (80 ml) in several portions over a period of 20 minutes with stirring at such a rate that the temperature did not exceed 0°. After 3 hours at 0° the reaction mixture, containing a red coloured precipitate, was brought to room temperature and, after bubbling nitrogen to remove the excess of nitrous acid, was filtered. The red-coloured precipitate was washed with water, then dissolved in chloroform. The chloroform solution, dried over magnesium sulphate and evaporated to small volume, yielded by addition of petroleum ether 4 g of the title compound as a red coloured powder. m.p. 179—180° (with decomposition) $[\alpha]_D^{23°} = +178°$ (c = 0.04 in $CHCl_3$). U.V. and VIS spectra: $\lambda_{max}^{MeOH}$ 233, 252, 290, 480, 496 and 530 nm ($E_{1cm}^{1\%}$ 643, 463, 156, 227, 232 and 134). Field desorption mass spectrum: m/e 510 ($M^+$) and 398. P.M.R. spectrum ($CDCl_3$): 1.38 (d, $CH_3$—C—4′), 2.43 (s, $CH_3CO$), 4.11 (s, $CH_3O$), 5.32 (broad s, C—7—H), 5.87 (broad s, C—1′—H). 7.2—8.1 (m, three aromatic protons), 9.78 (d, CHO), 13.16 and 13.89$\delta$ (two s, two phenolic protons). 13C—NMR (acetone-$d_6$): 16.6 ($CH_3$—C—4′); 24.1 (C—14); 33.1 (C—10); 33.3 (C—2′); 35.5 (C—8); 52.7 (C—3′); 56.4 (O—$CH_3$); 68.9 (C—7); 75.4 (C—4′); 76.7 (C—9); 106.2 (C—1′); 111.4 (C—5a, C—11a); 119.5 (C—4a, C—1, C—3); 134.7 (C—10a, C—12a); 135.3 (C—6a); 136.0 (C—2); 155.6 (C—11); 156.4 (C—6); 161.5 (C—4); 186.9 (C—5, C—12); 191.7 (CHO); 211.5 (C—13).

Example 2

Conversion of compound (III-a) into the corresponding 3′-epimer (III-b) (IMI—100).

A chloroform solution of compound III-a (2 g, in 20 ml) was chromatographed on a column of silica gel buffered at pH 7 (M/15 phosphate buffer) and elution with a 98:2 chloroform-acetone mixture gave pure compound III-b (1.5 g). Compound III-b can be distinguished from the starting compound (III-a) by thin layer chromatography (III-a) Rf 0.25 and III-b Rf 0.20 with 95:5 chloroform: acetone mixture) and by chemical and physical properties. m.p. 120° (with decomposition). $[\alpha]_D^{23°} = +246°$ (c=0.05 in MeOH). U.V. and VIS spectra $\lambda_{max}^{MeOH}$ 233, 252, 290, 480, 496 and 530 nm. Field desorption mass spectrum: m/e 510 ($M^+$) and 398. P.M.R. spectrum ($CDCl_3$): 1.40 (d, $CH_3$—C—4′), 2.46 (s, $CH_3CO$), 4.11 (s, $CH_3O$), 5.19 (broad s, C—7—H), 5.84 (broad s, C—1′—H), 7.2—8.1 (m, three aromatic protons), 9.71 $\delta$ (d, CHO), 13.14 and 13.94 $\delta$ (two s, two phenolic protons), 13C—NMR (acetone-$d_6$): 20.4 ($CH_3$—C—4′); 24.1 (C—14); 32.8 (C—10); 34.3 (C—2′); 35.8 (C—8); 56.4 (O—$CH_3$); 57.2 (C—3′); 68.5 (C—7); 74.3 (C—4′); 76.6 (C—9); 106.5 (C—1′); 111.2 (C—11a, C—5a); 119.4 (C—4a, C—1, C—3); 134.9 (C—10a, C—12a); 135.2 (C—6a); 136.0 (C—2); 155.6 (C—11); 156.6 (C—6); 161.5 (C—4); 186.4 (C—5); 186.8 (C—12); 201.8 (CHO); 211.8 (C—13).

Example 3

Preparation of 7-O-(2,3,5-trideoxy-3-C-formyl-$\alpha$-L-pentofuranosyl)-adriamycinone (III:$R_3$=OH), (IMI—99).

The preparation of the title compound starting from doxorubicin (II:$R_3$=OH) hydrochloride was performed according to the procedure described in Example 1.

The compound was obtained as a red coloured powder. m.p. 150° (with decomposition. $[\alpha]_D^{23°} = +269°$ (c, 0.05, in $CHCl_3$). U.V. and VIS spectra: $\lambda_{max}^{MeOH}$ 233, 253, 480, 496 and 530 nm ($E_{1cm}^{1\%}$ 520, 460, 222, 225 and 140). Field desorption mass spectrum: m/e 526 ($M^+$) and 414. P.M.R. spectrum ($CDCl_3$): 1.41 (d, $CH_3$—C—4′), 4.13 (s, $CH_3O$), 4.85 (broad s, $COCH_2$—O—), 5.38 (broad s, C—7—H), 5.87 (broad s, C—1—H), 7.2—8.1 (m, three aromatic protons), 9.80 (d, CHO), 13.01 and 13.96 $\delta$ (two s, two phenolic protons).

Example 4

Preparation of 7-O-(2,3,5-trideoxy-3-C-hydroxymethyl-$\alpha$-L-threopentofuranosyl)-daunomycinone (IMI.102).

A solution of compound III-a (510 mg, 1 mmol) in dioxan (80 ml) and aqueous acetate buffer at pH 4.6 (0.2 N, 20 ml) was portion-wise treated under stirring with sodium cyanoborohydride (500 mg, 8 mmol) at room temperature. After 3 hours the reaction mixture was diluted with water and extracted with chloroform. The extract was washed with water, dried over sodium sulphate, concentrated to small volume under reduced pressure then chromatographed on a column of silica gel buffered at pH 7 (M/15 phosphate buffer). Elution with a 99:1 chloroform:methanol mixture gave the title compound in pure form. m.p. 157—161° (with decomposition) $[\alpha]_n^{23°} = +286°$ (c=0.05, in MeOH). U.V. and VIS spectra: $\lambda_{max}^{MeOH}$ 234, 253, 290, 480, 496 and 530 nm ($E_{1cm}^{1\%}$ 600, 494, 118, 208, 212, 120). Field desorption mass spectrum: m/e 512 ($M^+$) and 398. 13C—NMR ($CDCl_3$): 122.13 (c—1); 139.26 (C—2); 123.38 (C—3); 164.65 (C—4); 190.03 (C—5); 160.12 (C—6); 72.09 (C—7); 40.13 (C—8); 80.70 (C—9); 36.90 (C—10); 159.51 (C—11); 190.27 (C—12); 216.37 (C—13); 28.59 (C—14); 60.34 ($OCH_3$—4); 124.45 (C—4a), 138.08, 138.46 (C—6a, C—10a, C—12a); 114.86, 114.77

(C—5a, C—11a); 109.50 (C—1'); 38.64 (C—2'); 46.11 (C—3'); 79.50 (C—4'); 19.65 (C—5'); 65.94 (C—6').

The title compound can be distinguished from the starting compound (III-a) also by thin layer chromatography eluting with a 4:1 chloroform: acetone mixture. III-a Rf=0.55, title compound Rf=0.25.

Example 5

Preparation of 7-C-(2,3,5-trideoxy-3-C-hydroxymethyl-$\alpha$-L-threopentafuranosyl)-daunomycinone (IMI 102)

A benzene suspension of sodium borohydride (8 equiv.) was refluxed with glacial acetic acid (6.5 equiv.) for 15 minutes under nitrogen to give a clear solution of sodium triacetoxyborohydride. To this solution was added a benzene solution of compound III-a (510 mg, 1mmol) and the mixture was refluxed for 1 hour. The reaction mixture was then diluted with water and extracted with chloroform. Work-up and column chromatographic purification as described in Example 4 were carried out, to give the title compound as characterised in Example 4. The yield was higher than in Example 4.

Example 6

Preparation of 7-O-(2,3,5-trideoxy-3-C-aminomethyl-$\alpha$-L-pentofuranosyl)-daunomycinone (V: $R_3$=H).

To a solution of compound III-a (1.02 g, 2 mmol) in dry dioxan (16 ml) was added a solution of ammonium acetate (1.32 g) in dry methanol (72 ml) and Molecular Sieve (4 g); the mixture was then treated with sodium cyanoborohydride (0.1 g). After three hours under stirring at room temperature the reaction mixture was diluted with water and the slightly acidic solution (pH 5.2) was extracted with chloroform in order to remove the starting material and some lipophilic impurities. The aqueous layer was brought to pH 7.2 with sodium hydrogen carbonate then extracted with chloroform and the extract after concentration was chromatographed on a column of silica gel buffered at pH 7 (M/15 phosphate buffer). Elution with a 89.5: 10: 0.5 chloroform: methanol: water mixture gave some fractions containing the title compound in pure form. The pooled fractions were diluted with water and the lower phase was washed with water, dried over anhydrous sodium sulphate and concentrated to a small volume under vacuum. Treatment with an equivalent of hydrochloric acid in methanol followed by precipitation with diethyl ether gave the title compound in pure form as the hydrochloride. m.p. 160°—165° (with decomposition). $[\alpha]_D^{23°} = +211°$ (c=0.02 m $CHCl_3$) $\lambda_{max}^{MeOH}$ 235, 254, 290, 480, 495 and 530 nm. Field desorption mass spectrum: m/e 511 ($M^+$), 398 and 362.

**Claims for the Contracting States: BE, CH, DE, FR, GB, IT, NL, SE**

1. Anthracycline glycosides of the general formula I

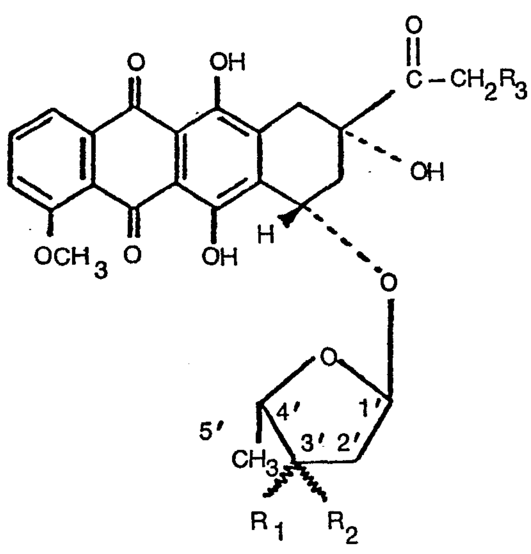

I

wherein one of $R_1$ and $R_2$ represents a hydrogen atom, the other of $R_1$ and $R_2$ represents a formyl, hydroxymethyl or aminomethyl group, and $R_3$ represents a hydrogen atom or a hydroxy or acyloxy group, and pharmaceutically acceptable acid addition salts of such compounds in which one of $R_1$ and $R_2$ represents an aminomethyl group.

2. 7-O-(2,3,5-trideoxy-3-C-formyl-$\alpha$-L-threopentafuranosyl)-daunomycinone (III-a).

3. 7-O-(2,3,5-trideoxy-3-C-formyl-$\alpha$-L-pentofuranosyl)-adriamycinone (III: $R_3$ = OH, $R_1$, $R_2$ = H, CHO).

4. 7-O-(2,3,5-trideoxy-3-C-hydroxymethyl-α-L-pentofuranosyl)-daunomycinone (IV: $R_3$ = H).

5. 7-O-(2,3,5-trideoxy-3-C-aminomethyl-α-L-pentofuranosyl)-daunomycinone (V: $R_3$ = H), or its hydrochloride.

6. A method for the preparation of an anthracycline glycoside according to claim 1, in which one of $R_1$ and $R_2$ represents a hydrogen atom and the other of $R_1$ and $R_2$ represents a formyl group, and $R_3$ represents hydrogen, hydroxy or acyloxy group, the method comprising reacting a compound of the general formula II

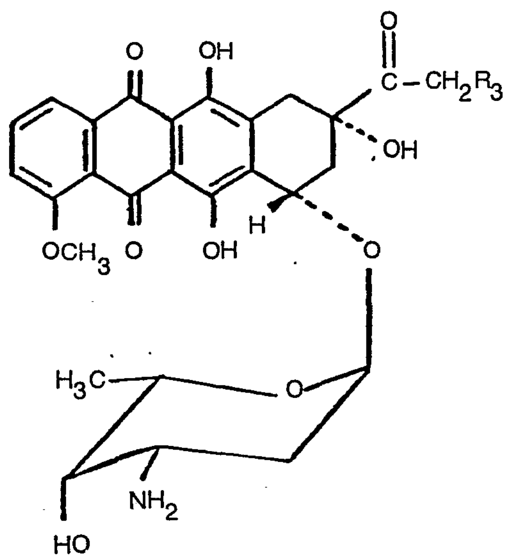

II

wherein $R_3$ has the meaning as described above, with sodium nitrite in a cold aqueous acidic medium.

7. A method according to claim 6 in which the aqueous acidic medium is 1N aqueous acetic acid.

8. A method according to claim 6 or claim 7 in which the reaction is carried out at 0°C.

9. A method for the preparation of an anthracycline glycoside according to claim 1 in which one of $R_1$ and $R_2$ represents a hydrogen atom and the other of $R_1$ and $R_2$ represents a hydroxymethyl group and $R_3$ represents a hydrogen atom or a hydroxy or acyloxy group, the method comprising selectively reducing an anthracycline glycoside prepared according to any of claims 6 to 8.

10. A method according to claim 9 in which the selective reduction is effected with sodium cyanoborohydride in an acidic buffer.

11. A method according to claim 10 in which the acidic buffer is a 4:1 mixture of dioxan: aqueous acetate at pH 4.6.

12. A method according to claim 9 in which the selective reduction is effected with sodium triacetoxyborohydride in benzene under reflux.

13. A method for the preparation of a compound according to claim 1 in which one of $R_1$ and $R_2$ represents a hydrogen atom and the other of $R_1$ and $R_2$ represents an aminomethyl group and $R_3$ represents a hydrogen atom or a hydroxy or acyloxy group, the method comprising selectively reductively aminating an anthracycline glycoside prepared according to any of claims 6 to 8 with sodium cyanoborohydride in the presence of methanolic ammonium acetate and a dehydrating agent.

14. Therapeutical composition with antitumor effect containing at least one of the compounds according to claim 1 together with usual pharmaceutically acceptable additives.

**Claims for the Contracting State: AT**

1. A method for the preparation of an anthracycline glycoside of the general formula I

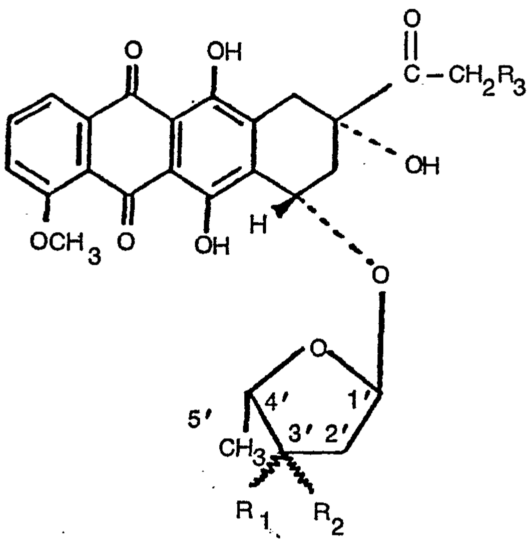

I

in which one of $R_1$ and $R_2$ represents a hydrogen atom and the other of $R_1$ and $R_2$ represents a formyl hydroxymethyl or aminomethyl group, and $R_3$ represents hydrogen, hydroxy or acyloxy group, the method comprising reacting a compound of the general formula II

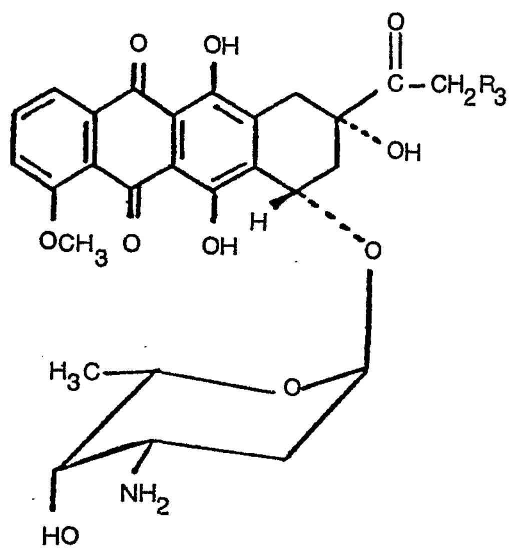

II

wherein $R_3$ has the meaning as described above, with sodium nitrite in a cold aqueous acidic medium and optionally selectively reducing or selectively reductively aminating a product thus prepared.

2. A method according to claim 1 in which the aqueous acidic medium is 1N aqueous acetic acid.

3. A method according to claim 1 or claim 2, in which the reaction is carried out at 0°C.

4. A method for the preparation of an anthracycline glycoside according to claim 1 in which one of $R_1$ and $R_2$ represents a hydrogen atom and the other of $R_1$ and $R_2$ represents a hydroxymethyl group and $R_3$ represents a hydrogen atom or a hydroxy or acyloxy group, the method comprising selectively reducing an anthracycline glycoside prepared according to any of claims 1 to 3.

5. A method according to claim 4 in which the selective reduction is effected with sodium cyanoborohydride in an acidic buffer.

6. A method according to claim 5 in which the acidic buffer is a 4:1 mixture of dioxan: aqueous acetate at pH 4.6.

7. A method according to claim 4 in which the selective reduction is effected with sodium triacetoxyborohydride in benzene under reflux.

8. A method for the preparation of a compound according to claim 1 in which one of $R_1$ and $R_2$ represents a hydrogen atom and the other of $R_1$ and $R_2$ represents an aminomethyl group and $R_3$ represents a hydrogen atom or a hydroxy or acyloxy group, the method comprising selectively reductively aminating an anthracycline glycoside prepared according to any of claims 1 to 3 with sodium cyanoborohydride in the presence of methanolic ammonium acetate and a dehydrating agent.

**Patentansprüche für die Vertragsstaaten: BE, CH, DE, FR, GB, IT, NL, SE**

1. Anthracyclinglykoside der allgemeinen Formel I

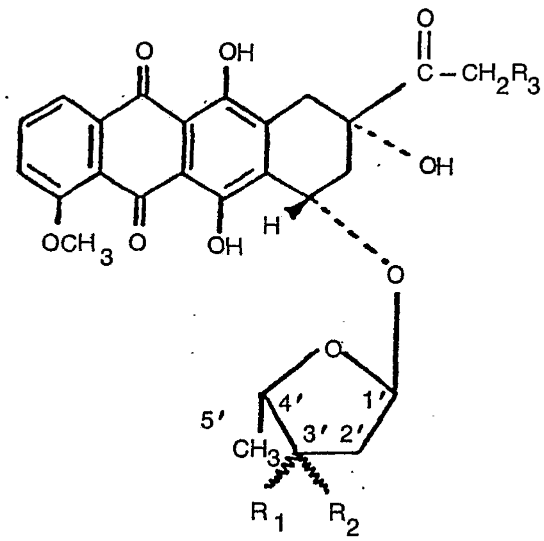

I

in welcher einer der Reste $R_1$ und $R_2$ ein Wasserstoffatom und der andere der Reste $R_1$ und $R_2$ eine Formyl-, Hydroxymethyl- oder Aminomethylgruppe und $R_3$ ein Wasserstoffatom oder eine Hydroxy- oder Acyloxygruppe bedeuten und pharmazeutisch annehmbare Säureadditionssalze solcher Verbindungen, bei denen einer der Reste $R_1$ oder $R_2$ eine Aminomethylgruppe bedeuten.

2. 7-O-(2,3,5-Trideoxy-3-C-formyl-$\alpha$-L-threopentafuranosyl)-daunomycinon (III-a).

3. 7-O-(2,3,5-Trideoxy-3-C-formyl-$\alpha$-L-pentofuranosyl)-adriamycinon (III: $R_3$ = OH, $R_1$, $R_2$ = H, CHO).

4. 7-O-(2,3,5-Trideoxy-3-C-hydroxymethyl-$\alpha$-L-pentofuranosyl)-daunomycinon (IV: $R_3$ = H).

5. 7-O-(2,3,5-Trideoxy-3-C-aminomethyl-$\alpha$-L-pentofuranosyl)-daunomycinon (V: $R_3$ = H) oder dessen Hydrochlorid.

6. Verfahren zur Herstellung eines Anthracyclinglykosids gemäß Anspruch 1, in denen einer der Reste $R_1$ und $R_2$ ein Wasserstoffatom und der andere $R_1$- und $R_2$-Rest eine Formylgruppe und $R_3$ ein Wasserstoffatom, eine Hydroxy- oder Acyloxygruppe bedeuten, dadurch gekennzeichnet, daß man eine Verbindung der allgemeinen Formel II

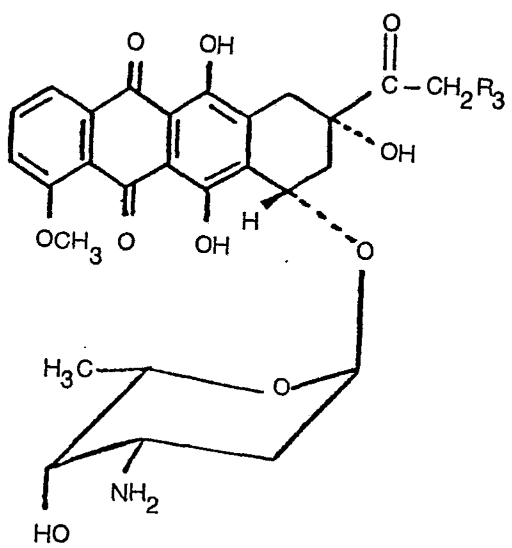

II

worin $R_3$ die vorgenannte Bedeutung hat, mit Natrium-nitrit in einem kalten wäßrigen sauren Medium umsetzt.

7. Verfahren gemäß Anspruch 6, dadurch gekennzeichnet, daß das wäßrige saure Medium 1N wäßrige Essigsäure ist.

8. Verfahren gemäß Anspruch 6 oder 7, dadurch gekennzeichnet, daß man die Umsetzung bei 0°C durchführt.

9. Verfahren zur Herstellung eines Anthracyclinglykosids gemäß Anspruch 1, bei denen einer der Reste $R_1$ und $R_2$ ein Wasserstoffatom und der andere Rest eine Hydroxymethylgruppe und $R_3$ ein Wasserstoffatom oder ein Hydroxy- oder Acyloxygruppe bedeuten, dadurch gekennzeichnet, daß man ein Anthracyclinglykosid, hergestellt gemäß einem der Ansprüche 6 bis 8 selektiv reduziert.

10. Verfahren gemäß Anspruch 9, dadurch gekennzeichnet, daß man die selektive Reduktion mit Natriumcyanoborohydrid in einem sauren Puffer durchführt.

11. Verfahren gemäß Anspruch 10, dadurch gekennzeichnet, daß der saure Puffer ein 4:1-Gemisch von Dioxan:wäßriges Acetat bei pH 4,6 ist.

12. Verfahren gemäß Anspruch 9, dadurch gekennzeichnet, daß man die selektive Reduktion mit Natriumtriacetoxyborohydrid in Benzol unter Rückfluß durchführt.

13. Verfahren zur Herstellung einer Verbindung gemäß Anspruch 1, in welcher einer der Reste $R_1$ und $R_2$ ein Wasserstoffatom und der andere Rest von $R_1$ und $R_2$ eine Aminomethylgruppe und $R_3$ ein Wasserstoffatom oder eine Hydroxy- oder Acyloxygruppe bedeuten, dadurch gekennzeichnet, daß man ein Anthracyclinglykosid, hergestellt nach einem der Ansprüche 6 bis 8, selektiv reduzierend mit Natriumcyanoborohydrid in Gegenwart von methanolischem Ammoniumacetat und einem Dehydratisierungsmittel aminiert.

14. Therapeutische Zusammensetzung mit Antitumorwirkung enthaltend wenigstens eine der Verbindungen gemäß Anspruch 1 zusammen mit üblichen pharmazeutisch annehmbaren Additiven.

**Patentansprüche für den Vertragsstaat: AT**

1. Verfahren zur Herstellung eines Anthracyclinglykosids der allgemeinen Formel I

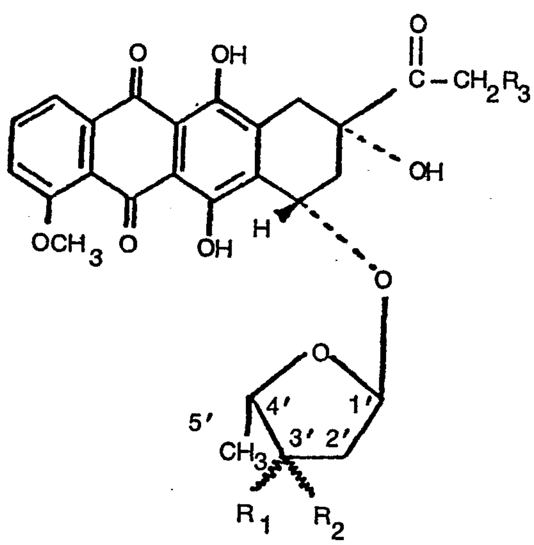

I

in welcher einer der Reste $R_1$ und $R_2$ ein Wasserstoffatom und der andere der Reste $R_1$ und $R_2$ eine Formyl-, Hydroxymethyl- oder Aminomethylgruppe und $R_3$ ein Wasserstoffatom oder eine Hydroxy- oder Acyloxygruppe bedeutet, dadurch gekennzeichnet, daß man eine Verbindung der allgemeinen Formel II

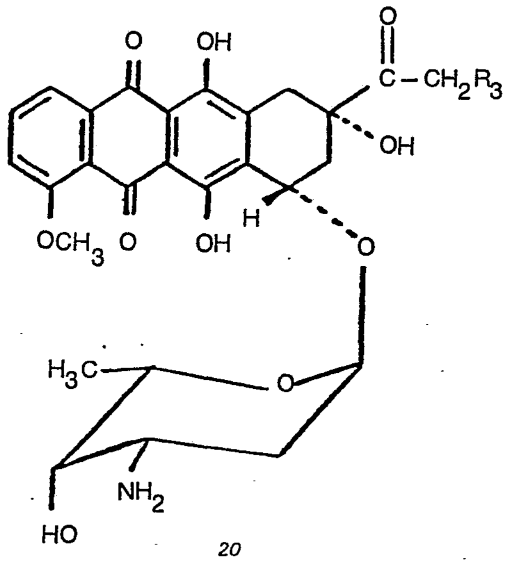 II

worin $R_3$ die vorgenannte Bedeutung hat, mit Natriumnitrit in einem kalten wäßrigen sauren Medium umsetzt und gegebenenfalls das so erhaltene Produkt selektiv reduziert oder selektiv reduzierend aminiert.

2. Verfahren gemäß Anspruch 1, dadurch gekennzeichnet, daß das wäßrige saure Medium 1N wäßrige Essigsäure ist.

3. Verfahren gemäß Anspruch 1 oder 2, dadurch gekennzeichnet, daß man die Umsetzung bei 0°C durchführt.

4. Verfahren zur Herstellung eines Anthracyclinglykosids gemäß Anspruch 1, bei denen einer der Reste $R_1$ und $R_2$ ein Wasserstoffatom und der andere Rest eine Hydroxymethylgruppe und $R_3$ ein Wasserstoffatom oder ein Hydroxy- oder Acyloxygruppe bedeuten, dadurch gekennzeichnet, daß man ein Anthracyclinglykosid, hergestellt gemäß einem der Ansprüche 1 bis 3 selektiv reduziert.

5. Verfahren gemäß Anspruch 4, dadurch gekennzeichnet, daß man die selektive Reduktion mit Natriumcyanoborohydrid in einem sauren Puffer durchführt.

6. Verfahren gemäß Anspruch 5, dadurch gekennzeichnet, daß der saure Puffer ein 4:1-Gemisch von Dioxan: wäßriges Acetat bei pH 4,6 ist.

7. Verfahren gemäß Anspruch 4, dadurch gekennzeichnet, daß man die selektive Reduktion mit Natriumtriacetoxyborohydrid in Benzol unter Rückfluß durchführt.

8. Verfahren zur Herstellung einer Verbindung gemäß Anspruch 1, in welcher einer der Reste $R_1$ und $R_2$ ein Wasserstoffatom und der andere Rest von $R_1$ und $R_2$ eine Aminomethylgruppe und $R_3$ ein Wasserstoffatom oder eine Hydroxy- oder Acyloxygruppe bedeuten, dadurch gekennzeichnet, daß man ein Anthracyclinglykosid, hergestellt nach einem der Ansprüche 1 bis 3, selektiv reduzierend mit Natriumcyanoborohydrid in Gegenwart von methanolischem Ammoniumacetat und einem Dehydratisierungsmittel aminiert.

**Revendications pour les Etats contractants: BE, CH, DE, FR, GB, IT, NL, SE**

1. Glycosides d'anthracycline de formule générale I:

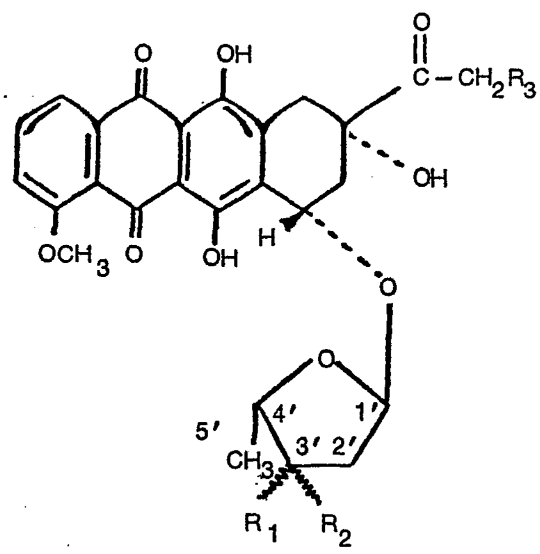

I

dans laquelle un des $R_1$ et $R_2$ représente un atome d'hydrogène, l'autre des $R_1$ et $R_2$ représente un groupe formyl, hydroxyméthyle ou aminométhyle; et $R_3$ représente un atome d'hydrogène ou un groupe hydroxyle ou acyloxy, et les sels acides d'addition pharmaceutiquement acceptables de ces composés dans lesquels un des $R_1$ et $R_2$ représente un groupe aminométhyle.

2. La 7-O-(2,3,5-tridésoxy-3-C-formyl-$\alpha$-L-thréopentafuranosyl)-daunomycinone (III-a).

3. La 7-O-(2,3,5-tridésoxy-3-C-formyl-$\alpha$-L-pentofuranosyl)-adriamycinone (III:$R_3$ = OH; $R_1$ = H, CHO).

4. La 7-O-(2,3,5-tridésoxy-3-C-hydroxyméthyl-$\alpha$-L-pentofuranosyl)-daunomycinone (IV:$R_3$ = H).

5. La 7-O-(2,3,5-tridésoxy-3-C-aminométhyl-$\alpha$-L-pentofuranosyl)-daunomycinone (V: $R_3$ = H), ou son chlorhydrate.

6. Procédé pour la préparation d'un glycoside d'anthracycline selon la revendication 1, dans lequel un des $R_1$ et $R_2$ représente un atome d'hydrogène, et l'autre des $R_1$ et $R_2$ représente un groupe formyl, et $R_3$ représente de l'hydrogène, un groupe hydroxyle ou acyloxy, le procédé comprenant la réaction d'un composé de formule générale II:

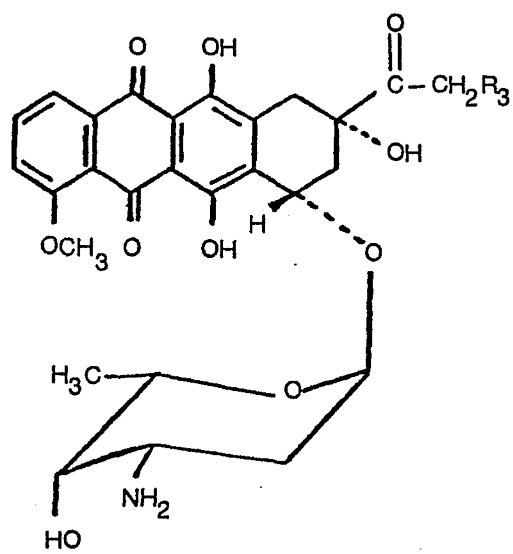

II

dans laquelle $R_3$ a la signification donnée ci-dessus, avec du nitrite de sodium dans un milieu acide aqueux froid.

7. Procédé selon la revendication 6, dans lequel le milieu acide aqueux est l'acide acétique aqueux 1N.

8. Procédé selon la revendication 6 ou la revendication 7, dans lequel la réaction est effectuée à 0°C.

9. Procédé pour la préparation d'un glycoside d'anthracycline selon la revendication 1, dans lequel un des $R_1$ et $R_2$ représente un atome d'hydrogène, et l'autre des $R_1$ et $R_2$ représente un groupe hydroxyméthyle, et $R_3$ représente un atome d'hydrogène ou un groupe hydroxyle ou acyloxy, le procédé comprenant la réduction sélective d'un glycoside d'anthracycline préparé selon l'une quelconque des revendications 6 à 8.

10. Procédé selon la revendication 9, dans lequel la réduction sélective est effectuée avec du cyanoborohydrure de sodium dans un tampon acide.

11. Procédé selon la revendication 10, dans lequel le tampon acide est un mélange 4:1 de dioxanne: acétate aqueux, à pH 4,6.

12. Procédé selon la revendication 9, dans lequel la réduction sélective est effectuée avec du triacétoxyborohydrure de sodium dans le benzène sous reflux.

13. Procédé pour la préparation d'un composé selon la revendication 1, dans lequel un des $R_1$ et $R_2$ représente un atome d'hydrogène et l'autre des $R_1$ et $R_2$ représente un groupe aminométhyle, et $R_3$ représente un atome d'hydrogène ou un groupe hydroxyle ou acyloxy, le procédé comprenant l'amination réductrice sélective d'un glycoside d'anthracycline préparé selon l'une quelconque des revendications 6 à 8 avec du cyanoborohydrure de sodium en présence d'acétate d'ammonium méthanolique et d'un agent de déshydratation.

14. Composition thérapeutique avec effet antimitotique contenant au moins un des composés selon la revendication 1 avec en même temps des additifs classiques pharmaceutiquement acceptables.

**Revendications pour l'Etat contractant: AT**

1. Procédé pour la préparation d'un glycoside d'anthracycline de formule générale I

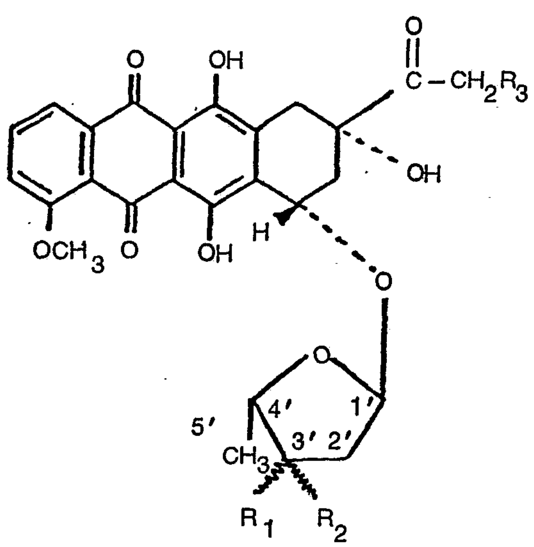

I

dans laquelle un des $R_1$ et $R_2$ représente un atome d'hydrogène, l'autre des $R_1$ et $R_2$ représente un groupe formyle, hydroxy-méthyle ou aminométhyle; et $R_3$ représente un atome d'hydrogène ou un groupe hydroxyle ou acyloxy, le procédé comprenant la réaction d'un composé de formule générale II

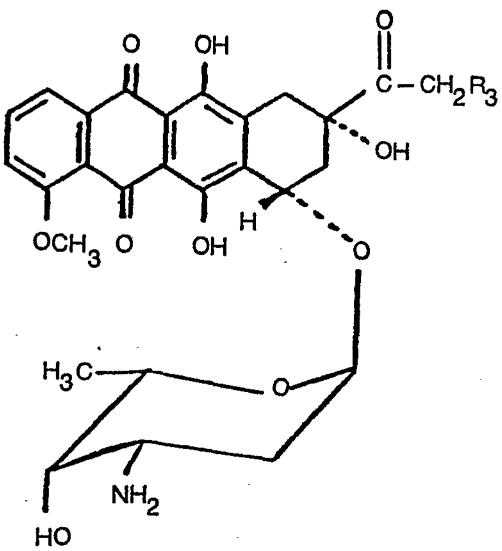

II

dans laquelle $R_3$ a la signification donnée ci-dessus, avec du nitrite de sodium dans un milieu acide aqueux froid, et le cas échéant on procédé par une réduction sélective ou une amination réductrice sélective du produit obtenu.

2. Procédé selon la revendication 1, dans lequel le milieu acide aqueux est l'acide acétique aqueux 1N.

3. Procédé selon la revendication 1 ou 2, dans lequel la réaction est effectuée à 0°C.

4. Procédé pour la préparation d'un glycoside d'anthracycline selon la revendication 1, dans lequel un des $R_1$ et $R_2$ représente un atome d'hydrogène, et l'autre des $R_1$ et $R_2$ représente un groupe hydroxyméthyle, et $R_3$ représente un atome d'hydrogène ou un groupe hydroxyle ou acyloxy, le procédé comprenant la réduction sélective d'un glycoside d'anthracycline préparé selon l'une quelconque des revendications 1 à 3.

5. Procédé selon la revendication 4, dans lequel la réduction sélective est effectuée avec du cyanoborohydrure de sodium dans un tampon acide.

6. Procédé selon la revendication 5, dans lequel le tampon acide est un mélange 4:1 de dioxanne: acétate aqueux, à ph 4,6.

7. Procédé selon la revendication 4, dans lequel la réduction sélective est effectuée avec du triacétoxyborohydrure de sodium dans le benzène sous reflux.

8. Procédé pour la préparation d'un composé selon la revendiation 1, dans lequel un des $R_1$ et $R_2$ représente un atome d'hydrogène et l'autre des $R_1$ et $R_2$ représente un groupe aminométhyle, et $R_3$ représente un atome d'hydrogène ou un groupe hydroxyle ou acyloxy, le procédé comprenant l'amination réductrice sélective d'un glycoside d'anthracycline préparé selon l'une quelconque des revendications 1 à 3 avec du cyanoborohydrure de sodium en présence d'acétate d'ammonium méthanolique et d'un agent de déshydratation.